# EUROPÄISCHE PATENTSCHRIFT

(11) **EP 1 273 266 B1**
(45) Veröffentlichungstag und Bekanntmachung des Hinweises auf die Patenterteilung: **14.09.2005**
(21) Anmeldenummer: 02012850.0
(22) Anmeldetag: 11.06.2002
(51) Int. Cl.: A61B 5/22, A63B 24/00

(54) **Verfahren zum Betrieb eines Trainingsgerätes nach Art eines Standfahrzeuges**
Method for operating a standing training device
Procédé de contrôle d'un appareil d'entraînement du type fixe

(30) Priorität: 07.07.2001 DE 10133053
(43) Veröffentlichungstag der Anmeldung: 08.01.2003
(73) Patentinhaber: Daum GmbH & Co. KG, 90587 Obermichelbach (DE)
(72) Erfinder: Daum, Wilhelm, 90768 Fürth (DE)
(74) Vertreter: Schneck, Herbert

(56) Entgegenhaltungen:
- EP-A- 0 569 879
- EP-A- 1 055 393
- WO-A-01/26535
- US-A- 4 278 095
- US-A- 4 708 337
- US-A- 4 911 427
- US-A- 5 230 673

## Beschreibung

Die Erfindung richtet sich auf ein Verfahren zum Betrieb eines Trainingsgeräts in Form eines Standfahrrads, wobei die Trainingsleistung der trainierenden Person durch Treten von Pedalen gegen eine gesteuerte Bremskraft aufgebracht wird, und wobei ein mit einer Speicher- und Recheneinrichtung verbundenes Graphikdisplay zur Anzeige von leistungsbezogenen Kenndaten und/oder zur Vorgabe von Trainingsprogrammen vorgesehen ist.

Die Bedeutung körperlicher Fitness hat im Bewußtsein einer breiten Öffentlichkeit in jüngerer Zeit erhebliche Bedeutung erlangt. In diesem Zusammenhang sind auch zahlreiche Heim-Trainer, z. B. in Form von Rudergeräten, insbesondere aber in Form von Standfahrrädern entwickelt worden, welche es nicht nur ermöglichen, zu Hause eine zeitliche und in ihre Intensität einstellbare körperliche Trainingsleistung zu erbringen, sondern welche auch mit Zusatzeinrichtungen ausgestattet worden sind, um wichtige körperliche Kenndaten über den Benutzer zu sammeln und dementsprechend Informationen über den körperlichen Zustand und Trainingsfortschritt zu geben, um dem Trainierenden einerseits ein Erfolgserlebnis durch einen erkennbaren, meßbaren Trainingsfortschritt zu geben und andererseits auch einen sportmedizinisch optimalen Trainingsaufbau zu ermöglichen.

So ist es beispielsweise bekannt, als Maßstab die körperliche Beanspruchung und die Erholungsrate nach Beendigung der körperlichen Beanspruchung die Pulsfrequenz der trainierenden Person zu messen. Ein derartiges Gerät ist beispielsweise in GB 1 593 839 B 1 beschrieben. Die US-PS 4,367,752 beschreibt eine Einrichtung zur Erfassung der Herzaktivität mit einer visuellen Anzeigeeinrichtung, welche insbesondere für das Training von Läufern im Freien bestimmt ist.

Die DE 39 08 756 A1 beschreibt eine Speichereinheit zur Steuerung eines Ergometers, welche die Datenaufnahme während des Trainings ermöglicht. Auch die DE-AS 26 30 293 beschreibt ein medizinisches Gerät mit einem Pulsaufnehmer, welches eine Auswertung der Trainingsleistung unter Berücksichtigung spezifischer Kenndaten der trainierenden Person ermöglicht. Ein Standfahrrad mit Pulsmeßgerät und Datenverarbeitung zur Optimierung des Trainingsaufbaus wird in der US-PS 4,911,427 beschrieben. Auch die DE 36 01 054 A1 und die DE 41 07 323 A1 beschreiben Geräte, die unter Berücksichtigung der Pulsaktivität eine Dosierung der körperlichen Belastung und eine Ausrichtung des Trainings ermöglichen.

Aus DE 93 07 352 U1 ist es bekannt, Sensoren zur Messung des Durchgangswiderstandes der Hauptoberfläche, d. h. des sogenannten psychogalvanischen Hauteffektes, vorzusehen, welche mit einem Mikroprozessor verbunden sind, wobei der Mikroprozessor seinerseits in Abhängigkeit von dem aus den Sensoren erhaltenen Signal akustische Anzeigegeräte steuert.

Die DE 299 11 700 beschreibt ein Trainingsgerät nach Art eines Standfahrrads mit einer Gewichtserfassungseinrichtung, durch welche das Gewicht der auf dem Sattel sitzenden oder einer stehenden trainierenden Person erfaßt wird, wobei weiterhin eine elektronische Auswerteeinrichtung vorgesehen ist, welche mit dem Ausgang der Gewichtserfassungseinrichtung verbunden ist und eine Anzeigeeinrichtung zur Anzeige des Gewichts umfaßt.

Die EP 0 569 879 A2 offenbart ein Trainingsfahrrad, bei dem der Benutzer zunächst eine Soll-Belastung und eine Ziel-Herzfrequenz vorgeben kann. Nach einer Trainingsaufwärmphase wird durch einen entsprechenden Sensor die Herzfrequenz des Benutzers gemessen und auf die Ziel-Herzfrequenz geregelt.

Die WO01/26535 A2 beschreibt ein Trainingsgerät mit einer Speicher- und Recheneinrichtung, welche Sensoren zur Messung verschiedener Körperparameter eines Benutzers aufweist.

Die US 4,278,095 A1 offenbart Beispiele für Trainingsprogramme, die auf einem Trainingsgerät abgearbeitet werden können.

Die EP 1 055 393 A2 offenbart ein Verfahren sowie eine Vorrichtung zur Ermittlung eines Körperparameters.

Hiervon ausgehend liegt der Erfindung die Aufgabe zugrunde, die bekannten Verfahren zum Trainieren mit Trainingsgeräten der in Betracht stehenden Art noch effektiver zu machen und weiter zu verbessern.

Diese Aufgabe wird erfindungsgemäß durch den Anspruch 1 gelöst.

Ein wichtiges Merkmal der Erfindung besteht also darin, daß nicht wie beim Stand der Technik einfach eine Vielzahl unterschiedlicher Trainingsprogramme angeboten werden, die die trainierende Person nach eigenem Gutdünhen auswählt, sondern daß eine Vorauswahl dahingehend stattfindet, was eigentlich das Trainingsziel ist und erst nach Tätigung einer solchen Vorauswahl speziell zur Realisierung des Trainingsziel geeignete Programme angeboten, demgegenüber andere ebenfalls vorhandene Programme aber nicht angeboten werden, wenn sie für die Erreichung des Trainingsziel nicht speziell geeignet sind.

In weiterer Ausgestaltung der Erfindung kann vorgesehen sein, daß bei Trainingsaufnahme das Gewicht und/oder der Körperfettgehalt der trainierenden Person erfaßt und abgespeichert werden, wobei bei nachfolgenden Trainingseinheiten ebenfalls eine derartige Erfassung und ein Vergleich mit den ursprünglich abgespeicherten Werten vorgenommen wird, und wobei das jeweilige Trainingsprogramm unter Berücksichtigung der Veränderung dieser Werte modifiziert wird.

Im Rahmen der Erfindung kann eine Mehrzahl von Fest-Programmen vorgesehen sein, wie leistungs- oder pulsgesteuerte Programme, Triathlon-, Rennstrecken-, Drehzahl-, Steigungs-, Kraft- und Auslauf-Programme.

Günstigerweise kann durch eine Niveauregulierungs-Einrichtung jedes individuelles Trainingsprogramm an die persönliche Leistungsfähigkeit angepaßt werden.

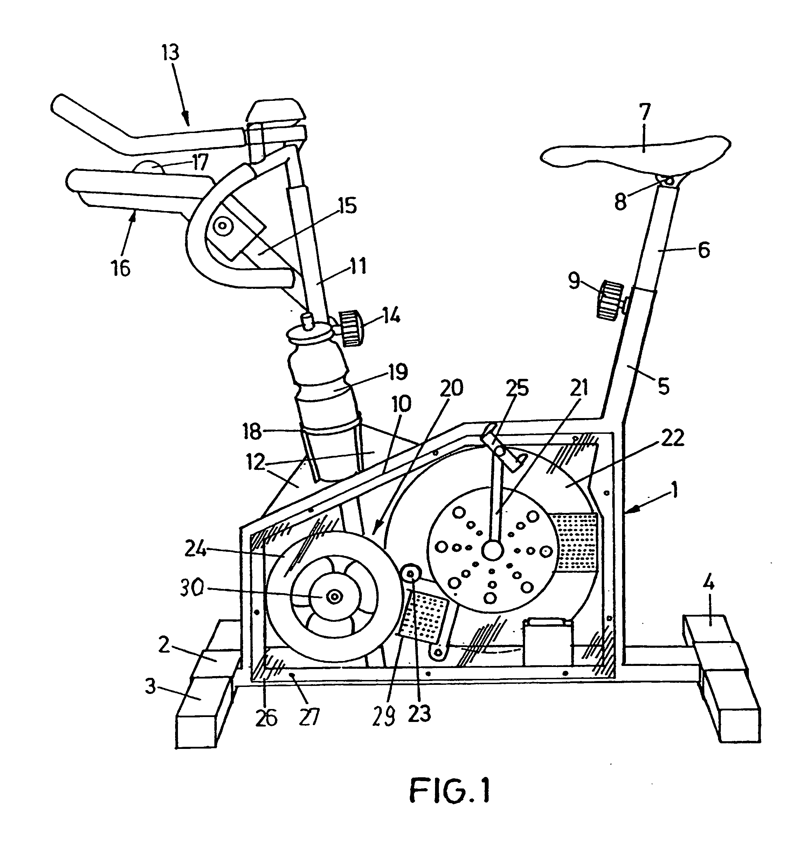

Nachfolgend wird die Erfindung anhand eines bevorzugten Ausführungsbeispiels in Verbindung mit der Zeichnung näher erläutert. Diese zeigt eine Ansicht eines erfindungsgemäßen Trainingsgerätes.

Ein in der Zeichnung dargestelltes Trainingsgerät umfaßt einen Rahmen 1 bestehend aus miteinander verschweißten Metallprofilen, wobei der Rahmen 1 über Klammerelemente 2 mit einem vorderen, quer verlaufenden Ständerteil 3 zu einem hinteren, quer verlaufenden Ständerteil 4 verbunden und dementsprechend gegen seitliches Umkippen gesichert ist.

Von dem hinteren Ende des Rahmens 1 erstreckt sich als Sattelaufnahme ein rechteckiges Stahlrohr 5 nach hinten oben. In dieses Stahlrohr 5 ist ein korrespondierendes Stahlrohr 6 teleskopartig eingesetzt. An dem oberen Ende dieses zweiten Stahlrohres 6 ist ein Sattel 7 über eine in der Zeichnung im einzelnen nicht dargestellte Einrichtung 8 nach vorne und hinten positionseinstellbar auswechselbar befestigt. Über eine Feststellschraube 9 kann die Verschiebbarkeit des Stahlrohrs 6 relativ zum Stahlrohr 5 unterbunden werden, das heißt eine Höheneinstellung vorgenommen werden.

Ausgehend von einem oberen, schräg nach vorne verlaufenden Rahmenteil 10 des Rahmens 1 erstreckt sich nach oben ein als Lenkeraufnahme dienendes Stahlrohr 11, welches über Haltewinkel 12 abgestützt ist.

In dem Stahlrohr 11 ist ein handelsüblicher Triathlonlenker 13 über eine Schraube 14 höhenverstellbar festgelegt.

Von dem unteren Abschnitt des Stahlrohrs 11 erstreckt sich schräg nach oben ein Träger 15, an dessen oberem Ende eine Meßanzeigeeinrichtung 16 angeordnet ist. Diese umfaßt einen Dreh-Einstellknopf 17, der vom Lenker 13 aus ohne Verlassen der Fahrposition betätigbar ist.

An dem Stahlrohr 11 ist ein Halter 18 für zwei Getränkeflaschen 19 angeordnet.

Im Inneren des Rahmens 1 ist ein Riemengetriebe 20 angeordnet, umfassend eine Tretkurbel 21, welche mit einem Schwungrad 22 in Verbindung steht und einen um eine Umlenkrolle 23 geführten Treibriemen, der um eine Scheibe 24 der Wirbelstrombremse geführt ist.

An der Tretkurbel sind Pedale 25 auswechselbar derart befestigt, daß der Rennfahrer ihm gewohnte übliche Fahrpedale anbringen kann. Der Rahmen 1 ist seitlich mit transparenten Abdeckplatten 26 aus Acrylglas abgedeckt, welche über Distanzschrauben 27 auf Abstand zum Rahmen 1 gehalten werden, so daß entstehende Wärmeenergie entweichen kann.

Die Wirbelstrombremse umfaßt einen Elektromagnet 29. Die Scheibe 24 ist über ein Kugellager 23 gelagert. Zur Kraftübertragung ist eine Riemenspannanordnung eingeschaltet, welche eine Umlenkrolle 23 umfaßt.

Im Bereich der Feststellschraube 9 ist eine Gewichtserfassungseinrichtung in Form eines Druckaufnehmers angeordnet. Um gleichwohl eine Höhenverstellung realisieren zu können, ist in dem Stahlrohr 5 ein Stahlrohrstumpf mit geschlossener Unterseite angeordnet, der an seiner Außenseite Bohrungen oder Rillen aufweist, in die die Feststellschraube 9 zur Höhenverstellung eingreift. Das Stahlrohr 6, an welchem der Sattel 7 befestigt ist, greift mit axialem Spiel in diesen Rohrstumpf ein und drückt mit seiner unteren Stirnseite auf die geschlossene Unterseite des Rohrstumpfes, wo ein elektronischer Druckaufnehmer angeordnet ist, der einen Druck erfaßt, der dem Gewicht des Sattels 7 zuzüglich des Gewichtes der auf dem Sattel 7 sitzenden Person proportional ist. Der Ausgang dieses elektronischen Druckaufnehmers ist mit der Auswerteeinrichtung bzw. Meßanzeigeeinrichtung 16 verbunden, so daß dort ein Wert entsprechend dem Gewicht der trainierenden Person angezeigt wird und über eine Datenverarbeitungseinrichtung anhand dieses so ermittelten Gewichts spezielle Trainingsprogramme vorgegeben werden können.

Ein vorstehend beschriebenes Trainingsgerät wird erfindungsgemäß wie folgt betrieben:

Betätigt der Benutzer während des Anzeigemodus der Gesamtkilometer die als Taste ausgebildete Auswahleinrichtung, so erscheint am Graphikdisplay eine Anzeige zur Auswahl der Trainingsziele, wobei automatisch die zuletzt gefahrene Trainingsart markiert ist. Die Auswahleinrichtung umfaßt auch ein Jogg-Shuttle, mittels dessen der Benutzer die gewünschte Trainingsart auswählt und dies durch Drücken des Drehknopfes bestätigt.

Hat der Benutzer sein gewünschtes Trainingsziel bestätigt, so erscheint auf dem Bildschirm eines Auswahl der Trainingswochen. Es ist dabei immer die zum aktuellen Tages-Datum gehörende Kalenderwoche markiert. Prozent-Angaben stellen dabei das Verhältnis zwischen den in der jeweiligen Woche absolvierten Trainingseinheiten zu der vom Benutzer gewählten Trainingshäufigkeit dar.

Bestätigt der Benutzer die markierte Trainingswoche, so erscheint auf dem Bildschirm eine Übersicht "Trainingseinheiten", wobei mittels des Jogg-Shuttles auf eine niedrigere Woche umgeschaltet werden kann.

Unter einer Anzeige "Trainingseinheit" erscheinen nur so viele Trainingseinheiten, wie der Benutzer zuvor ausgewählt hat. Bestätigt der Benutzer die markierte Trainingseinheit, so beginnt das Training mit der zum jeweiligen Trainingsprogramm passenden Anzeige.

Mittels des Jogg-Shuttles kann aber auch auf eine niedrige Trainingseinheit umgeschaltet werden.

Die Trainings-Steuerung ist an ein im Cockpit vorgesehenes Zeitmodul gekoppelt und erfolgt entsprechend dem aktuellen Datum.

Wird ein neues Trainingsprogramm begonnen, wird die Übereinstimmung des zeitlichen Ablaufs mit dem vorgegebenen Programm überprüft und bei Einhaltung des Zeitplans eine jeweils abgearbeitete Woche freigegeben.

Wird die Vorgabe nicht eingehalten, wird der Benutzer über das Display aufgefordert, zusätzliche Trainingseinheiten zu absolvieren.

In regelmäßigen Abständen während des zeitlichen Verlaufes des Trainingsprogramms, beispielsweise alle vier Wochen, findet ein Leistungstest statt, der die aktuellen Benutzerparameter zur Leistungseinstufung feststellt Als Testprofil wird ein Programm verwendet, bei welchem die Leistung stufenweise gesteigert wird, wobei jede Stufe zwei Minuten beansprucht. Der Test endet entweder, wenn die Trainingsperson aufhört zu treten und daraufhin eine Trainings-Taste betätigt, oder wenn die gemessene Ist-Herzfrequenz die maximale, vorgegebene Soll-Herzfrequenz erreicht. Während des Tests wird außerdem der von der Trainingsperson maximal erreichte Pulswert entsprechend der Ist-Herzfrequenz gespeichert. Die gemessenen Werte werden über einen Algorithmus mit einem Faktor entsprechend der persönlichen Leistungseinstufung des Benutzers modifiziert.

Je nach ausgewählten Trainingsziel, z. B. Herz-Kreislauf-Training oder Gewichtsreduzierung, erfolgt eine individuell zugeschnittene Programmsteuerung.

Das Herz-Kreislauf-Training stellt vom Anspruch her beispielsweise das Minimal-Programm dar und belastet die Trainingsperson am wenigsten. Zielgruppe sind dabei noch gesunde Anfänger ohne Trainingserfahrung. Ziel ist der Aufbau eines leistungsfähigen Herz-Kreislauf-Systems und die Prophylaxe vor Herzerkrankungen und Arteriosklerose. Der Trainingsumfang beträgt drei bis fünf Einheiten wöchentlich. Durch die Ermittlung eines Kardio-Pulses anstatt eines Ausdauer-Pulses besteht ein weiter Sicherheitsbereich für die Herzfrequenzvorgaben.

Demgegenüber unterscheidet sich beispielsweise das Programm zur Gewichtsreduzierung hiervon. Zur Gewichtsreduzierung ist ein sehr niedrigintensives pulsgesteuertes Programm sinnvoll mit einem Trainingsumfang von 3 bis 7 Einheiten wöchentlich, wobei bestimmte leistungsgesteuerte Fest-Programme nicht zugelassen werden. Mit einem solchen Programm kann im ersten Monat eine Gewichtsreduzierung um etwa 4 kg realistischerweise erreicht werden, wobei danach zirka 700 g wöchentlich erzielbar sind.

Für die Trainingsart Muskelaufbau-Training wird in der Regel der Widerstand im Trainingsprozeß erhöht, wobei hier drehzahlgesteuerte Bremsstufen-Programme zum Einsatz kommen. Der Trainingsumfang beträgt hier 3 bis 7 Einheiten wöchentlich.

## Patentansprüche

1. Verfahren zum Betrieb eines Trainingsgeräts in Form eines Standfahrrads, wobei die Trainingsleistung der trainierenden Person durch Treten von Pedalen gegen eine gesteuerte Bremskraft aufgebracht wird, wobei ein mit einer Speicher- und Recheneinrichtung verbundenes Graphikdisplay zur Anzeige von leistungsbezogenen Kenndaten und/oder zur Vorgabe von Trainingsprogrammen vorgesehen ist, mit folgendem Schritt:
- Betätigung einer Auswahleinrichtung zur Auswahl der Trainingsart bzw. des Trainingsziels, wie Herz-Kreislauf-Training, Gewichts- und /oder Fettreduzierung, Konditionstraining, Muskelaufbau o. dgl.,
**gekennzeichnet durch** die weiteren Schritte:
- Vorauswahl von speziellen Trainingsprogrammen aus einer Mehrzahl vorhandener Trainingsprogramme anhand des ausgewählten Trainingsziels,
- Durchführung eines Fitness-Tests nach Vorgabe auf dem Graphikdisplay, wobei die Trainingsleistung erfasst und mit im Speicher des Rechners abgelegten Kenndaten verglichen wird und anschließend über das Graphikdisplay ein Fitness-Ist-Zustand angezeigt wird und
- Berechnung eines auf den Fitness-Ist-Zustand der trainierenden Person und die gewünschte Trainingsart zugeschnittenen Trainingsprogramms aus den vorausgewählten speziellen Trainingsprogrammen.

2. Verfahren nach Anspruch 1, **dadurch gekennzeichnet, daß** bei Trainingsaufnahme das Gewicht und/oder der Körperfettgehalt der trainierenden Person erfaßt und abgespeichert werden, wobei bei nachfolgenden Trainingseinheiten ebenfalls eine derartige Erfassung und ein Vergleich mit den ursprünglich abgespeicherten Werten vorgenommen wird, und wobei das jeweilige Trainingsprogramm unter Berücksichtigung der Veränderung dieser Werte modifiziert wird.

3. Verfahren nach Anspruch 1, **dadurch gekennzeichnet, daß** eine Mehrzahl von Festprogrammen, wie z. B. leistungs- oder pulsgesteuerte Programme, Triathlon-, Rennstrecken-, Tretzahl-, Steigungs-, Kraft- und Auslauf-Programme, abgespeichert sind.

4. Verfahren nach Anspruch 3, **dadurch gekennzeichnet, daß** jedes individuelles Trainingsprogramm durch eine Niveauregulierungs-Einrichtung an die persönliche Leistungsfähigkeit anpaßbar ist.

## Claims

1. A method of operating an exerciser in the form of a home bike, with the performance of an exercising person being mustered by the person pedalling against a controlled braking power, with a graphics display that is connected to a storage and computing unit being provided for indicating performance characteristics and/or for setting exercise programs, comprising the following step:
- actuation of a device for selecting the type and target of exercise, such as exercise of the heart and circulatory system, weight and/or fat reduction, fitness training, muscle build-up or the like;
**characterized by** the further steps:
- pre-selection of special exercise programs from a plurality of available exercise programs based on the selected target;
- realization of a fitness test after rate setting on the graphics display, with the workout being detected and compared to the characteristics deposited in the memory of the computer and with an actual state of fitness then being indicated by the graphics display; and
- computation, from the pre-selected specific exercise programs, of an exercise program suited to the actual state of fitness of the exercising person and the desired type of exercise.

2. A method according to claim 1, **characterized in that**, at the beginning of the exercise, the weight and/or body fat component of the person exercising is detected and stored, with that kind of detection also taking place during ensuing exercise units and a comparison to the values originally stored taking place, and with the respective exercise program being modified based on the changes of these values.

3. A method according to claim 1, **characterized in that** a plurality of fixed programs are stored, such as for example performance- or pulse-controlled programs, triathlon-, track-, pedalling-rate-, strength- and cooling-down-programs.

4. A method according to claim 3, **characterized in that** each individual exercise program is adaptable to the individual fitness by a level-adjusting device.

## Revendications

1. Procédé pour faire fonctionner un appareil d'exercice sous forme d'une bicyclette fixe, dans lequel la puissance d'exercice de la personne effectuant l'exercice est exercée en appuyant sur les pédales à l'encontre d'une force de freinage contrôlée, un affichage graphique relié à un dispositif de mémorisation et de calcul étant prévu pour afficher les caractéristiques relatives à la puissance et/ou pour prédéfinir des programmes d'exercice, avec l'étape suivante :
- actionnement d'un dispositif de sélection pour sélectionner le type d'exercice ou l'objectif de l'exercice, comme par exemple l'entraînement de la circulation cardiaque, la perte de poids et/ou de graisse, la remise en forme, la musculation ou d'autres objectifs du même genre,
**caractérisé par** les autres étapes suivantes :
- présélection de programmes d'exercice spéciaux parmi une pluralité de programmes d'exercice existants à l'aide de l'objectif sélectionné,
- réalisation d'un test de condition physique après une prédéfinition sur l'affichage graphique, la puissance d'exercice étant détectée et comparée avec des caractéristiques mémorisées dans l'ordinateur, puis une condition physique réelle étant affichée sur l'affichage graphique et
- calcul d'un programme d'exercice adapté à la condition physique réelle de la personne effectuant l'exercice et au type d'exercice souhaité à partir des programmes d'exercice spéciaux présélectionnés.

2. Procédé selon la revendication 1, **caractérisé en ce que**, pendant l'enregistrement de l'exercice, le poids et/ou la masse graisseuse de la personne effectuant l'exercice sont détectés et mémorisés, une détection similaire et une comparaison avec les valeurs mémorisées initialement étant également effectuées pendant les séquences d'exercice suivantes et le programme d'exercice concerné étant modifié pour tenir compte de la variation de ces valeurs.

3. Procédé selon la revendication 1, **caractérisé en ce qu'**une pluralité de programmes fixes, comme par exemple des programmes contrôlés par la puissance ou par le pouls, des programmes de triathlon, de parcours de course, de nombre pédalages, de montée, de force et de roue libre, est mémorisée.

4. Procédé selon la revendication 3, **caractérisé en ce que** chaque programme d'exercice individuel peut être adapté à la capacité personnelle au moyen d'un dispositif de régulation de niveau.
